# EUROPÄISCHE PATENTSCHRIFT

(11) **EP 1 228 203 B1**
(45) Veröffentlichungstag und Bekanntmachung des Hinweises auf die Patenterteilung: **18.06.2008**
(21) Anmeldenummer: 00987221.9
(22) Anmeldetag: 08.11.2000
(51) Int. Cl.: C12N 15/11, C12N 15/12, C07K 7/08, C07K 14/435, C12N 15/62, C12N 15/63, C07K 16/40, A61K 38/10, A61P 31/18

(54) **HUMANES ZIRKULIERENDES VIRUS INHIBIERENDES PEPTID (VIRIP) UND SEINE VERWENDUNG**
PEPTIDE (VIRIP) WHICH INHIBITS A CIRCULATING VIRUS IN HUMANS AND THE USE THEREOF
PEPTIDE INHIBITEUR DE VIRUS CIRCULANT CHEZ L'HOMME (VIRIP) ET SON UTILISATION

(30) Priorität: 08.11.1999 DE 19953732; 16.05.2000 DE 10023665
(43) Veröffentlichungstag der Anmeldung: 07.08.2002
(73) Patentinhaber: IPF Pharmaceuticals GmbH, 30625 Hannover (DE)
(72) Erfinder: KIRCHHOFF, Frank, 89092 Ulm (DE); MÜNCH, Jan, 89231 Neu-Ulm (DE); STÄNDKER, Ludger, 30625 Hannover (DE); FORSSMANN, Wolf-Georg;, 30625 Hannover (DE); ADERMANN, Knut;, 30177 Hannover (DE)
(74) Vertreter: Meyers, Hans-Wilhelm
(86) Internationale Anmeldenummer: PCT/EP2000/011020
(87) Internationale Veröffentlichungsnummer: WO 2001/034640

(56) Entgegenhaltungen:
- WO-A-84/02918
- WO-A-97/46100
- US-A- 5 420 110
- SHAPIRO LELAND ET AL: "Alpha-1-antitrypsin inhibits human immunodeficiency virus type 1." FASEB JOURNAL, Bd. 15, Nr. 1, Januar 2001 (2001-01), Seiten 115-122, XP002164955 ISSN: 0892-6638

## Beschreibung

Gegenstand der vorliegenden Erfindung ist ein Polypeptid (Eiweißstoff) gemäß Anspruch 1 und dessen Verwendung gemäß Anspruch 2 mit inhibierenden Eigenschaften auf die virale Infektion von Zellen: Humanes Virus inhibierendes Peptid (VIRIP) und seine therapeutische und diagnostische Verwendung. Die Erfindung umfasst die natürlich vorkommende Form de VIRIP sowie abgeleitete Fragmente und/oder Analoga bzw. Derivate gemäß den Patentansprüchen sowie schließlich ein Arzneimittel enthaltend die natürlichen, rekombinanten und synthetischen Peptide zur Verwendung für medizinische Indikationen und zur Verwendung als ein Diagnosemittel. Die Erfindung umfasst darüber hinaus modifizierte Formen des VIRIP gemäß Anspruch 1, die eine besonders günstige therapeutische Wirksamkeit aufweisen. Des weiteren eines seiner Fragmente und/oder Derivate zu diagnostischen oder therapeutischen Zwecken, insbesondere bei viralen Erkrankungen und zur Behandlung von Infektionen mit HIV-1 und HIV-2.

Die VIRIP konnte überraschenderweise mit Hilfe von chromatographischen Methoden und mit Hilfe eines biologischen Assays aus humanem Hämofiltrat isoliert werden. Die biochemische Charakterisierung des erfindungsgemäßen Peptids erfolgte durch Massenspektrometrie und vollständigen Sequenzierung aller Aminosäuren.

Das Peptid besitzt die folgende Aminosäuresequenz:
LEAIPMSIPPEVKFNKPFVF

Die Molekularmasse des erfindungsgemäßen Peptids VIRIP beträgt 2303 Da, wenn Z₁ und Z₂ keine Aminosäurereste bedeuten.

Als Derivate des VIRIP sind amidierte, acetylierte, sulfatierte, Polyethylenglycol (PEG) modifizierte, phosphorylierte und/oder glykosylierte Derivate, welche die Infektion und/oder Replikation von bzw. in humanen Blutzellen durch HIV-1 unterdrücken, zu nennen.

Das erfindungsgemäße Peptid umfasst ein 20 Aminosäuren umfassendes Fragment des bekannten humanen Proteins Alpha-1-Antitrypsin (Accession No. P01009), welches in seiner prozessierten Form aus 394 Aminosäuren besteht. Die Funktion des Alpha-1-Antitrypsin wird vornehmlich als Hemmstoff für die Enzyme Elastase sowie Thrombin und Plasmin beschrieben. Die erfindungsgemäße Peptidsequenz des VIRIP beginnt vorzugsweise hinter der Aminosäure 352 des Alpha-1-Antitrypsin und umfasst somit die Aminosäuren 353 bis 372 des Alpha-1-Antitrypsin.

Überraschenderweise bewirkt das erfindungsgemäße Peptid eine Unterdrückung der HIV-1-Infektion und/oder -Replikation von bzw. in humanen Blutzellen.

Das erfindungsgemäße Peptid ist durch ein Reinigungsverfahren ausgehend von humanem Hämofiltrat erhältlich. Dieses Hämofiltrat fällt bei der Ultrafiltration des Blutes von Nierenkranken in großen Mengen an.

Gegenstand der Erfindung sind auch Polynukleotide, die für das erfindungsgemäße Peptid kodieren, wobei diese Polynukleotide bevorzugt aus DNA-RNA genomischer DNA oder PNA aufgebaut sind. Ein weiterer Aspekt der Erfindung sind Vektoren, die die erfindungsgemäßen Polynukleotide enthalten sowie gentechnisch manipulierte Wirtszellen, die den erfindungsgemäßen Vektor enthalten.

Das erfindungsgemäße Peptid kann auch mit einem Adapterprotein gekoppelt sein, das die Aufnahme in virusinfizierbare Zellen gewährleistet.

Gegenstand der Erfindung sind auch Verwendungen zur Herstellung von Arzneimitteln zur Behandlung von Patienten, die VIRIP benötigen durch Gabe therapeutischer Mengen der erfindungsgemäßen Polypeptide.

Alternativ kann die therapeutische Wirkung des erfindungsgemäßen Polypeptides durch Gabe von Polynukleotiden kodierend für VIRIP und anschließende Expression in vivo beim Patienten erreicht werden.

Das humane Hämofiltrat wird gegebenenfalls mit Wasser verdünnt und angesäuert. Der pH-Wert beträgt vorzugsweise 1,5 bis 3,5, insbesondere 2,5 bis 3,0. Danach wird das Hämofiltrat über einen Kationenaustauscher geleitet, beispielsweise einem mit Sulfonsäuregruppen modifizierenden Trägermaterial (Fraktogel SP - 650 (M), Merck, Darmstadt). Die an den Kationenaustauscher gebundenen Peptide werden mit einer relativ hoch konzentrierten Salzlösung eluiert. Die Ionenstärke der Elutionslösung entspricht dabei ungefähr einer 0,5 bis 1 molaren Ammoniumacetatlösung.

Das aufgefangene Eluat wird einer weiteren Kationenaustauscher-Chromatographie unterzogen. Diese Chromatographie ist vorzugsweise eine Stufenelution mit Puffern von ansteigenden pH-Werten.

Die das erfindungsgemäße Peptid enthaltenen Fraktionen werden mittels präparativer Umkehrphasen-Chromatographie und nachfolgender semipräparativer Umkehrphasen-Chromatographie beispielsweise an mit C18 modifizierten Trägermaterialien weitergereinigt. Der Aufreinigungsgrad wird vorzugsweise mittels analytischer Umkehrphasen-Chromatographie beispielsweise an mit C18 modifizierten Trägermaterialien überprüft.

Die durch die chromatographische Reinigung erhaltene Substanz wurde der Strukturaufklärung zugeführt. Die Bestimmung der Molekülmassen des gereinigten Peptids erfolgte mittels eines Elektrospray Massenspektrometers (ESI-MS). Die Sequenzanalyse des nativen Peptids erfolgte über einen Edman-Abbau mit einem ABI 473 A Sequenzer. Die erfindungsgemäße Peptidsequenz wurde chemisch synthetisiert und die Struktur des synthetisch hergestellten Peptids wurde ebenfalls aufgeklärt. Dieses synthetisch hergestellte VIRIP bewirkt ebenfalls eine dosis-abhängige Unterdrückung der HIV-1-Infektion und/oder -Replikation von bzw. in humanen Blutzellen.

Das erfindungsgemäße Peptid sowie seine cDNA, sein Gen und Analoga; Fragmente und Derivate zu dem Peptid, der cDNA und dem Gen können als Arzneimittel Verwendung finden. Seine biologische Aktivität entspricht der virus-inhibierender Substanzen. Dabei kann vermutet werden, dass VIRIP aufgrund seiner kurzen, hydrophoben Sequenz in die Blutzellen aufgenommen wird und dort als Hemmstoff von Virus-Enzymen oder als Hemmstoff von Enzymen der Blutzellen wirkt oder dass VIRIP an Rezeptoren bindet, welche für den Eintritt von Viren von Bedeutung sind. Somit verhindert VIRIP die Infektion von Zellen mit dem Virus.

Das erfindungsgemäße Peptid kann dabei in für Peptide üblicher Weise parenteral, intravenös, intramuskulär, intranasal, lokal-topisch, subkutan oder bukal verabreicht werden. Die Menge an zu verabreichendem Peptid beträgt 1 mg bis 1 g pro Darreichungseinheit pro Tag. Die Wirkung des erfindungsgemäßen Peptids kann durch Gabe geeigneter Inhibitoren/Antagonisten gehemmt werden.

Die Antikörper gegen ein erfindungsgemäßes Peptid können durch Immunisierung von Säugetieren erhalten werden. Das erfindungsgemäße Diagnostikmittel enthält DNA, RNA und/oder PNA gegebenenfalls in modifizierter und/oder markierter Form zum Einsatz in dem Fachmann bekannten Testsystemen wie PCR oder Fingerprinting.

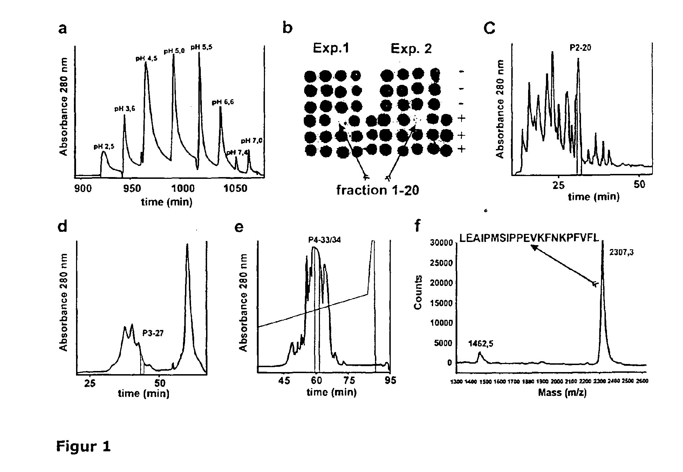

Figur 1: Aufreinigung von VIRIP. Die Aufreinigungsschritte sind im Text beschrieben. (b) Inhibition der Replikation von HIV-1 NL43 in humanen Blutlymphozten durch die Fraktion 1-20. Die Zellen wurden 2 Tage mit PHA stimuliert, mit Virusstocks die 10 ng p24 enthalten infiziert und die Reverse Transkriptase-Aktivität im Kulturüberstand 9 Tage nach Infektion bestimmt. Dargestellt ist das Ergebnis der Infektionsexperimente.

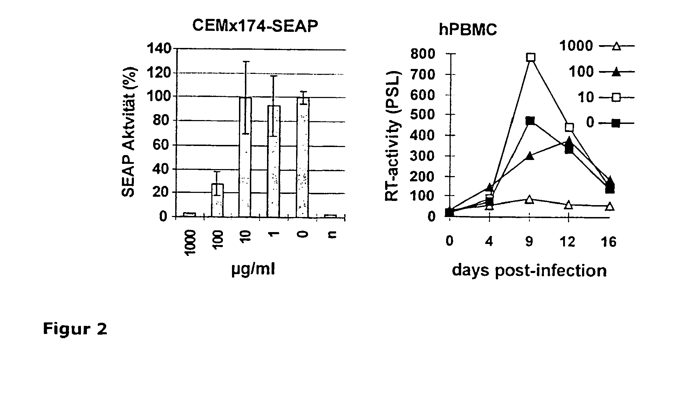

Figur 2: Einfluss von synthethischem VIRIP auf die Infektion von CEMx174-SEAP Indikatorzellen (links) und die Replikation in humanen PBMCs (rechts). Die Zellen wurden in Gegenwart der aufgeführten Mengen an VIRIP infiziert und kultiviert. Zur Bestimmung des viralen Eintritts wurde die Aktivität der sekretierten Alkalinen Phosphatase in Kulturüberstand der CEMx174-SEAP Zellen drei Tage nach der Infektion bestimmt.

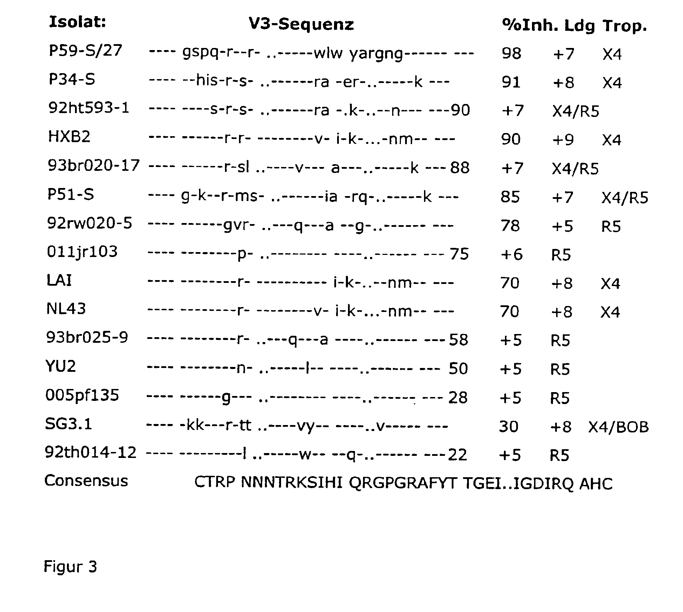

Figur 3: Die V3-Schleife des X4-tropen NL43 Klons wurde gegen die entsprechenden Sequenzen der dargestellten HIV-1 Isolate ausgetauscht. Die rekombinanten Viren unterscheiden sich somit ausschließlich in der V3-Schleife. Im rechten Panel ist der beobachtete inhibitorische Effekt (%), die Gesamtladung der V3-Region und der Korezeptortropismus dargestellt.

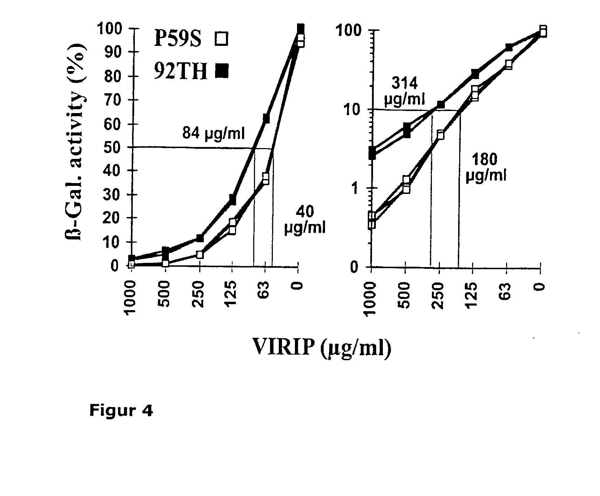

Figur 4: Dosis-abhängige Hemmung der X4-tropen P59S und der R5-topen 92TH HIV-1 NL4-3 Rekombinanten durch VIRIP. P4R5 Indikatorzellen wurden in Gegenwart der dargestellten Konzentrationen an VIRIP infiziert und die β-Galaktosidase-Aktivität im Zellextrakt 2 Tage nach Infektion gemessen.

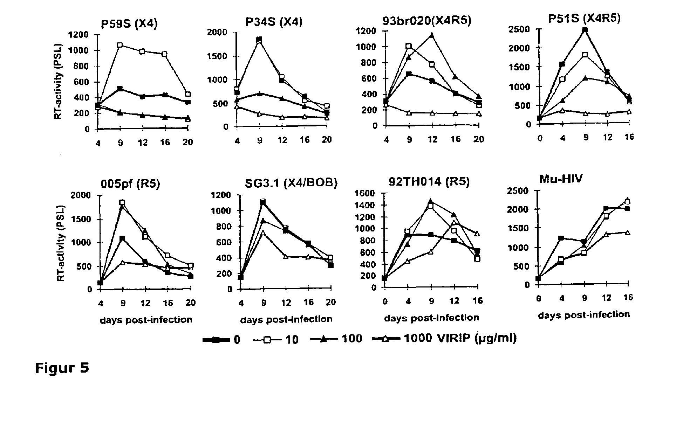

Figur 5: VIRIP hemmt die Vermehrung verschiedener HIV-Isolate mit unterschiedlichem Zell- und Korezeptor-Tropismus in humanen PBMC. Vorstimulierte PBMC wurden in Gegenwart der dargestellten VIRIP Konzentrationen mit den diversen HIV-1 Isolaten (10 ng p27 antigen) oder einem chimären Onko-Lentivirus (Mu-HIV). Die Virusvermehrung wurde durch die Messung der Reverse Transkriptase-Aktivität im Zellkulturüberstand bestimmt.

Die Erfindung wird anhand der folgenden Beispiele näher beschrieben.

### Beispiel 1

### Isolierung des antiviral wirksamen VIRIP

### 1. Schritt: Hämofiltrat-Batch-Extraktion

800-1000 L Hämofiltrat werden mit HCI auf einen pH -Wert von 2.7 eingestellt und mit Wasser auf eine Leitfähigkeit von 5.5 mS/cm verdünnt und mit einer Flussrate von 3 L/min auf einen starken Kationenaustauscher aufgetragen.

### Chromatographiebedingungen:

- Säule:: Vantage VA 250 (Amicon, Witten)
- Säulenmaterial:: Fractogel TSK SP 650 (M), 25 cm x 20 cm
- Fluss:: 3 L/min
- Detektion:: 280 nm, pH, Leitfähigkeit
- Puffer A:: Hämofiltrat pH 2.7, Leitfähigkeit 5.5 mS/cm
- Puffer B:: 0.5 M Ammoniumacetat
- Anlage:: Autopilot Chromatographiesystem, (PerSeptive Bio systems, Wiesbaden)

Nach Auftrag der insgesamt 1.000 L Flüssigkeit über Nacht wird mit mehreren Säulenvolumina 5 mM HCl gespült. Die Elution der gebundenen Peptide erfolgt als Batch-Elution mit 0.5 M Ammoniumacetat. Hierbei wird eine komplette Elution der Peptide über steigenden pH-Wert (6.8 - 7.2) und steigende Leitfähigkeit (56 mS/cm) in etwa 5 L Eluat erreicht.

### 2. Schritt: Erste präparative Auftrennung (Charge 01/1998)

Die Ammoniumacetat-Eluate der Batch-Extraktion werden in einer Menge von 10.000 L Hämofiltrat-Peptid vereinigt. Nach pH-Einstellung auf 2.7 wird das Peptidextrakt unter Zumischung von VE-Wasser mit einer Leitfähigkeit von 5.5 mS/cm auf den präparativen Kationenaustauscher aufgetragen.

### Chromatographiebedingungen:

Säule: Vantage 250 VA
Säulenmaterial: Fractogel TSK SP 650 (M), 25 cm x 20 cm
Fluss: bis zu 3 L/min während des Auftrages 0.5 bis 1 L während der Elution
Detektion: 280 nm, pH, Leitfähigkeit
Probe: Hämofiltrat pH 2.7, Leitfähigkeit 5.5 mS/cm
Anlage: Autopilot Chromatographiesystem, (PerSeptive Bio systems, Wiesbaden)

Nach Auftrag des Rohextraktes über 240 min wird die Säule mit 0.01 M HCl gespült, bis die Leitfähigkeit unter 1 mS/cm ist. Die Elution erfolgt dabei in mehreren Stufen mit den im folgenden angegebenen Puffern

| Puffer | pH-Wert | Puffersubstanzen | Leitfähigkeit (mS/cm) |
|---|---|---|---|
| Waschpuffer: | 2.0 | 0.01 M HCI | 1 |
| Elutionspuffer 1: | 3.6 | 0.1 M Zitronensäure-1-hydrat | 2.9 |
| Elutionspuffer 2: | 4.5 | 0.1 M Essigsäure + 0.1 M Natriumacetat | 4.0 |
| Elutionspuffer 3: | 5.0 | 0.1 M Äpfelsäure | 6.2 |
| Elutionspuffer 4: | 5.6 | 0.1 M Bernsteinsäure | 6.1 |
| Elutionspuffer 5: | 6.6 | 0.1 M NaH₂PO₄ | 4.9 |
| Elutionspuffer 6: | 7.4 | 0.1M NaH₂PO₄ | 6.7 |
| Elutionspuffer 7: | 9.0 | 0.1 M Ammoniumcarbonat | 6.7 |

Die Eluate 1-7 werden als pH-Pool I-VII bezeichnet (s. Fig. 1a). Sie werden separat gesammelt und abschließend mit VE-Wasser gespült. Die Elution erfolgt bis zum Erreichen einer neuen Basislinie, wobei für die einzelnen pH-Pools I bis VII Elutionsvolumina von 10 bis 25 L erreicht werden.

### 3. Schritt: Zweite präparative Auftrennung:

Die einzelnen pH-Pools werden zur Fraktionierung und gleichzeitigen Entsalzung über eine Reversed Phase Chromatographie getrennt

### Chromatographiebedingungen:

- Säule:: FineLine 100 (Pharmacia, Freiburg)
- Säulenmaterial:: Source RPC, 15 µm 10 x 12.5 cm (FineLine 100)
- Fluss:: 150 mL/min (FineLine 100)
- Detektion:: 280 nm, Leitfähigkeit, pH
- Puffer A:: 10 mM HCl
- Puffer B:: 80% Acetonitril in 10 mM HCl
- Gradient:: 0-60% Puffer B in 5 Säulenvolumen

Nach Auftrag der einzelnen pH-Pools wird die Säule mit Puffer A gespült. Während der Elution werden Fraktionen zu 200 ml gesammelt. Die Fraktionen werden gefriergetrocknet und bei -20°C gelagert. Aliquots der entstandenen Fraktionen werden im Bioassay getestet. Die Fraktion 20 aus pH-Pool I enthielt das erfindungsgemäße Peptid (s. Fig. 1b, c).

### 4. Schritt: Semipräparative Reverse-Phase C18-Chromatographie:

Insgesamt 500 mg der im Assay bioaktiven Fraktion 20 aus pH-Pool I wurden über eine semipräparative Reverse-Phase Säule aufgetrennt. Die Fraktion 27 enthielt die erfindungsgemäße Substanz (s. Fig. 1d).

### Chromatographiebedingungen:

- Säule:: 4,7 cm x 30 cm Stahlsäule
- Füllmaterial:: Vydac RP-C18 15-20 µm, 300 Å
- Puffer A:: 30% Methanol, 70% Wasser, 0.1% TFA
- Puffer B:: 100% Methanol, 0.1% TFA
- Gradient:: 0 - 60% B in 2100 ml
- Fluss:: 40 ml/min
- Detektion:: 214 nm und 280 nm
- Chromatographieanlage:: BioCad 250, Perseptive Biosystems
- Fraktionen:: á 50 ml ab Start des Gradienten

### 5. Schritt: Analytische Reverse-Phase C4-Chromatographie:

Die bioaktive Fraktion 27 aus der vorhergehenden Chromatographie wurde über eine analytische Reverse-Phase Säule aufgetrennt. Aliquots wurden im Bioassay getestet. Die Fraktionen 33+34 enthielten die erfindungsgemäße Substanz in aufgereinigter Form.

*Chromatographiebedingungen:* 5 µm, 100 Å, 20 x 250 mm;
- Säule:: 2 cm x 25 cm Stahlsäule
- Füllmaterial:: RP-C4, 5 µm, 100 Å, Biotek Silica, Östringen, Germany
- Puffer A:: Wasser, 0.1 % TFA
- Puffer B:: 80 % Acetonitril, 20% Wasser, 0.1 % TFA,
- Gradient:: 20 - 60% B in 80 min, 60 - 100% B in 2 min
- Fluss:: 8 ml/min
- Detektion:: 214 nm und 280 nm
- Chromatographieanlage:: Kontron
- Fraktionen:: á 1.5 min ab Start des Gradienten

Die erfindungsgemäße Reinsubstanz wurde daraufhin in dosisabhängiger Weise im Bioassay untersucht und peptidchemisch charakterisiert.

### Beispiel 2

### Massenbestimmungen

Die Massenbestimmungen des aus Hämofiltrat isolierten Peptids (aus den Fraktionen 33+34 des 5. Schritts in Beispiel 1) und des chemisch synthetisierten Peptids (Beispiel 3) wurden auf einem ESI Massenspektrometer durchgeführt (s. Fig. 1f). Die Molekülmasse der Peptide wurden entsprechend der nachfolgend gezeigten Massenzahlen (MW) bestimmt:
- VIRIP, isioliert aus humanem Hämofiltrat:: 2303 Da
- VIRIP, chemisch synthetisiertes Peptid:: 2303 Da

### Sequenzbestimmung

Die aufgereinigten nativen und chemisch synthetisierten Peptide werden mittels Edman-Abbau auf einem ABI 473 A Sequenzer unter Verwendung des Standard-Programms analysiert. Die Proben werden auf eine Polybrene-Membran in Mengen zwischen 100 und 400 pmol aufgetragen. Es ergaben sich sowohl für das aus Hämofiltrat isolierte Peptid (aus den Fraktionen 33+34 des 5. Schritts in Beispiel 1) und das chemisch synthetisierte Peptid (Beispiel 3) folgende identische vollständige Aminosäuresequenz:
LEAIPMSIPPEVKFNKPFVF

### Datenbankvergleich

Ein Datenbankvergleich wurde mit Hilfe des HUSAR-Programmpakets an den SwissProt und EMBL-Nukleinsäure Datenbanken durchgeführt. Die Peptidsequenz besitzt eine hundertprozentige Identität zu dem aus der cDNA abgeleiteten Aminosäuren 353-372 des humanen Proteins Alpha-1-Antitrypsin (Accession No. P01009).

### Beispiel 3

### Chemische Synthese von VIRIP

Die chemische Synthese von VIRIP wurde mittels konventioneller Festphasensynthese auf einem Peptid-Synthesizer 9050 unter Verwendung der bekannten Fmoc-Chemie durchgeführt. Das erhaltene Peptid wurde über Reverse-Phase Chromatographie aufgereinigt, seine Identität und Reinheit wurde mittels analytischer RP-HPLC sowie der unter Beispiel 2 beschriebenen Massen- und Sequenzbestimmung festgestellt.

### Beispiel 4

### Bestimmung der antiviralen Aktivität von VIRIP

Die Isolierung der VIRIP erfolgte aufgrund ihrer inhibitorischen Wirkung auf die Vermehrung von HIV-1 in einem Assay, der die Replikation von HIV-1 in peripheren humanen Blutlymphozyten (PBMCs) misst. Dazu wurden jeweils Aliquots der unter Beispiel 1 beschriebenen einzelnen Chromatographiestufen gefriergetrocknet und anschließend dem biologischen Assay in Mengen von 10 ml bis zu 1 L Hämofiltrat-Äquivalent zugeführt. Die Fraktionen, die jeweils ein positives Signal ergaben, wurden der weiteren Aufreinigung unterzogen.

Humane periphere Blutlymphozyten (PBMCs) wurden aus Vollblut mittels Dichtegradienten-Zentrifugation in Ficoll gewonnen. Die Zellen wurden für zwei Tage vorstimuliert (RPMI-Medium, 20% FKS, 100 U/ml IL-2, 5 µg/ml Phytohämagglutinin [PHA]). Anschließend wurden die PBMCs durch Zentrifugation für 5 min bei 1200 rpm sedimentiert, in PHA-freiem RPMI-Medium aufgenommen (20% FKS, 100 U/ml IL-2) und in einer Konzentration von etwa 150.000 Zellen pro Loch in 96-Well-Flachboden-Platten in einem Volumen von 80 µl ausgesät.

Am folgenden Tag wurden Aliquots der unter Beispiel 1 beschriebenen einzelnen Chromatographiestufen in Mengen von 10 ml bis zu 1 L Hämofiltrat-Äquivalent zugeführt. Dazu wurden die gefriergetrockneten Chromatographiestufen in Wasser aufgenommen und in verschiedenen Konzentrationen in einem Gesamtvolumen von 10 µl zu den PBMCs pipettiert. Nach 2stündiger Inkubation bei 37°C wurden die Zellen durch die Zugabe von 10 µl HIV-1 Virusstock, der 0,1 bis 10 ng p24 Antigen enthält, infiziert. Im folgenden wurden 50 µl des Kulturmediums alle 2 Tage durch frisches Medium ersetzt, welches zusätzlich die korrespondierenden Peptid-Fraktionen enthält. Die Produktion an HIV-Partikeln zu verschiedenen Zeitpunkten (9, 12 und 18 Tage) nach der Infektion der Zellen wurde im Reverse-Transkriptase(RT)-Test quantifiziert. Der RT-Test misst die Aktivität der Reversen-Transkriptase, im zellfreien Kulturüberstand und ist somit ein Maß für die Produktion an Viruspartikeln und die virale Vermehrung in den PBMC-Kulturen. Alternativ wurde die Virusproduktion im p24 Antigen-ELISA bestimmt. Fraktionen, die im Vergleich zu Ansätzen ohne potentiell stimulierende oder inhibitorische Peptide zu einer mindestens 90%igen Reduktion der RT-Produktion führten (s. z.B. Fig. 1b) und somit die Vermehrung von HIV-1 in humanen PBMC effizient hemmten, wurden weiter aufgereinigt.

Das aus Hämofiltrat aufgereinigte VIRIP (Beispiel 1) als auch das chemisch synthetisierte VIRIP (Beispiel 3) zeigten eine dosisabhängige Inhibition der HIV-1 Infektion von CEM×174-SEAP Indikatorzellen und der Replikation in PBMCs (Fig. 2). Das erfindungsgemäße VIRIP besitzt dagegen keine zytotoxische Wirkung auf die Blutzellen.

### Beispiel 5. Die Effizienz der VIRIP-spezifischen Inhibition ist abhängig von der Sequenz der V3-Schleife im HIV-1 Hüllprotein.

Die Ergebnisse belegten, dass sowohl VIRIP aus Hämofiltrat, als auch das synthetisch hergestellte Peptid die Replikation von HIV-1 NL43 wirksam blokkieren. Um herauszufinden, ob VIRIP spezifisch für X4-trope Varianten ist oder auch andere Formen inhibiert, welche den Korezeptor CCR5 benutzen oder dual-trop (X4/R5) sind, wurde eine Reihe von V3-Varianten des molekularen NL4-3 Klons untersucht (Fig. 3). Der V3-Loop des X4-tropen NL4-3 Klons wurde durch die entsprechende Region einer Reihe von primären HIV-1 Isolaten ersetzt. Funktionelle Tests mit verschiedenen Indikatorzellinien zeigten, dass diese V3-Rekombinanten einen unterschiedlichen Zell- und Korezeptor-Tropismus aufweisen. Um den inhibitorischen Einfluss von VIRIP auf diese Varianten zu untersuchen wurden P4R5 Indikatorzellen verwendet. P4R5-Zellen exprimieren sowohl CCR5 als auch CXCR4 und enthalten das Luziferasegen unter der Kontrolle der HIV-1 LTR. Diese Zellinie wurde in Gegenwart und Abwesenheit von VIRIP mit den diversen rekombinanten Viren infiziert (50 ng p24) und die Infektion mit Hilfe des Luziferasetest quantifiziert. Wie in der Figur 3 dargestellt inhibierte VIRIP sowohl X4-, als auch R5- und X4/R5-trope Varianten. Insgesamt wurden jedoch X4-trope Varianten mit stark positiv geladenem V3-Loop effizienter gehemmt als R5- oder dual-trope Isolate.

Um den inhibitorischen Effekt der erfindungsgemäßen Substanz genauer zu quantifizieren, wurden P4R5 Indikator Zellen in Gegenwart verschiedener Dosen von VIRIP mit dem X4-tropen HIV-1 P59S und dem R5-tropen 92TH Isolat infiziert. Wir in der Fig. 4 dargestellt, inhibierte VIRIP das HIV-1 P59S Isolat bei einer Konzentration von 40 µg/ml um 50%, und bei einer Konzentration von 180 µg/ml um 90%. Zur Hemmung des X-tropen 92TH Isolates waren etwa 2-fach höhere Konzentrationen an VIRIP erforderlich (Fig. 4).

In weiteren Experimenten wurde gezeigt, dass VIRIP die Replikation von X4 - tropen und dual-tropen HIV-Varianten in humanen PBMC bei Konzentrationen von 1000 µg/ml komplett und bei Konzentrationen von 100 µg/ml zumindest partiell unterdrückt (Fig. 5, oben). Die inhibitorischen Effekte auf R5-trope Formen waren geringer. Ein chimäres Onko-Lentivirus (Mu-HIV), welches das MLV-Hüllprotein trägt, wurde nicht inhibiert (Fig. 5, unten).

## Patentansprüche

1. Peptid (VIRIP) mit nachfolgender Aminosäuresequenz:
LEAIPMSIPPEVKFNKPFVF
sowie Fragmente des Peptids und/oder amidierte, acetylierte, sulfatierte, Polyethylenglycol (PEG) modifizierte, phosphorylierte und/oder glykosylierte Derivate des Peptids, welche die Infektion und/oder Replikation von bzw. in humanen Blutzellen durch HIV-1 unterdrücken.

2. Verwendung eines Peptids mit nachfolgender Aminosäuresequenz:
Z₁-LEAlPMSIPPEVKFNKPFVF-Z₂ (VIRIP)
und/oder amidierten, acetylierten, sulfatierten, Polyethylenglycol (PEG) modifizierten, phosphorylierten und/oder glykosylierten Derivaten des Peptids, welche die Infektion und/oder Replikation von bzw. in humanen Blutzellen durch HIV-1 unterdrücken,
darin repräsentieren Z₁, Z₂ unabhängig voneinander eine Anzahl von 0 bis zu 10 Aminosäureresten, und falls Z, oder Z₂=0 Aminosäurereste bedeuten, dann Z₁=H und/oder Z₂=COOH,
zur Herstellung eines Arzneimittels zur Behandlung von Infektionen mit HIV-1 und HIV-2.

3. Polynukleotide kodierend für das Peptid oder dessen Fragmente nach Anspruch 1.

4. Polynukleotide nach Anspruch 3, **dadurch gekennzeichnet, dass** die Polynukleotide aus DNA, RNA, genomischer DNA oder PNA aufgebaut sind.

5. Vektor enthaltend die Polynukleotide aus Anspruch 3.

6. Gentechnisch manipulierte Wirtszelle enthaltend den Vektor nach Anspruch 5.

7. Peptide aus Anspruch 1 in Verbindung mit einem Adapterprotein, das die Aufnahme in Virus-infizierbare Zellen gewährleistet.

8. Galenische Formulierung bestehend aus Peptiden nach Anspruch 1 oder 7 und einem verträglichen Carrier.

9. Verfahren zur Herstellung des Peptides gemäß Anspruch 1 durch Extraktion von Hämofiltrat durch Kationenaustauscher-Extraktion mit nachfolgender Elution der adsorbierten Substanzen, eine erneute Kationenaustauscher-Chromatographie des die Peptide enthaltenden Extraktes sowie mehrstufige Umkehrphasen-Chromatographie.

10. Verfahren zur Herstellung des Peptides gemäß Anspruch 1 durch Festphasensynthese im Sinne der Merrifield-Synthese sowie Flüssigphasensynthese nach dem Fachmann bekannten Methoden mit geschützten Aminosäuren und dessen Aufreinigung.

11. Verfahren zur Herstellung des Peptides gemäß Anspruch 1 durch dem Fachmann bekannte Verfahren der heterologen Expression mittels gängiger biotechnologischer Vektoren.

12. Diagnostikmittel enthaltend Polynukleotide nach Anspruch 3 oder 4.

13. Arzneimittel enthaltend die Peptide nach Anspruch 1 oder die Polynukleotide nach Anspruch 3 oder 4 als wirksamen Bestandteil von galenischen Formen zur oralen, intravenösen, intramuskulären, intracutanen, subcutanen, intrathekalen Anwendung sowie als Aerosol zur transpulmonalen Applikation.

14. Verwendung der Polynukleotide nach Anspruch 3 oder 4 zur Herstellung eines Arzneimittels zur Behandlung von Infektionen durch HIV-1 oder HIV-2.

## Claims

1. A peptide (VIRIP) having the following amino acid sequence:
LEAIPMSIPPEVKFNKPFVF
and fragments of said peptide and/or amidated, acetylated, sulfated, polyethylene glycol (PEG) modified, phosphorylated and/or glycosylated derivatives of said peptide which suppress the infection of human blood cells by HIV-1 and/or replication thereof in such cells.

2. Use of a peptide having the following amino acid sequence:
Z₁-LEAIPMSIPPEVKFNKPFVF-Z₂ (VIRIP)
and/or amidated, acetylated, sulfated, polyethylene glycol (PEG) modified, phosphorylated and/or glycosylated derivatives of said peptide which suppress the infection of human blood cells by HIV-1 and/or replication thereof in such cells;
wherein Z₁ and Z₂ are independently a sequence of from 0 to 10 amino acid residues, and if Z₁ or Z₂ = zero amino acid residues, then Z₁ = H and/or Z₂ = COOH;
for the preparation of a medicament for the treatment of infections by HIV-1 and HIV-2.

3. Polynucleotides coding for the peptide or its fragments according to claim 1.

4. The polynucleotides according to claim 3, **characterized by** being constituted of DNA, RNA, genomic DNA or PNA.

5. A vector containing the polynucleotides according to claim 3.

6. A genetically engineered host cell containing the vector according to claim 5.

7. The peptides according to claim 1 in connection with an adaptor protein which ensures uptake into virus-infectable cells.

8. A galenic formulation consisting of peptides according to claim 1 or 7 and a compatible carrier.

9. A process for the preparation of the peptide according to claim 1 by extraction from hemofiltrate by cation-exchange extraction followed by elution of the adsorbed substances, renewed cation-exchange chromatography of the extract containing the peptides, and multi-step reverse-phase chromatography.

10. A process for the preparation of the peptide according to claim 1 by solid-phase synthesis in terms of Merrifield synthesis or liquid-phase synthesis by methods known to the skilled person using protected amino acids, and its purification.

11. A process for the preparation of the peptide according to claim 1 by methods of heterologous expression known to the skilled person using common biotechnological vectors.

12. A diagnostic agent containing the polynucleotides according to claim 3 or 4.

13. A medicament containing the peptides according to claim 1 or the polynucleotides according to claim 3 or 4 as an active ingredient of galenic forms for oral, intravenous, intramuscular, intracutaneous, subcutaneous, intrathecal administration, and as an aerosol for transpulmonary administration.

14. Use of the polynucleotides according to claim 3 or 4 for the preparation of a medicament for the treatment of infections by HIV-1 or HIV-2.

## Revendications

1. Peptide (VIRIP) ayant la séquence d'acides aminés suivante :
LEAIPMSIPPEVKFNKPFVF
ainsi que fragments du peptide et/ou dérivés amidés, acétylés, sulfatés, modifiés par du polyéthylèneglycol (PEG), phosphorylés et/ou glycosylés du peptide, qui inhibent 1'infection et/ou la réplication de ou dans des cellules sanguines humaines par le VIH-1.

2. Utilisation d'un peptide ayant la séquence d'acides aminés suivante :
Z₁-LEAIPMSIPPZVKFNKPFVF-Z₂ (VIRIP)
et/ou de dérivés amidés, acétylés, sulfatés, modifiés par du polyéthylèneglycol (PEG), phosphorylés et/ou glycosylés du peptide, qui inhibent l'infection et/ou la réplication de ou dans des cellules sanguines humaines par le VIH-1,
séquence dans laquelle Z₁, Z₂ représentent, indépendamment l'un de l'autre, un nombre de 0 à 10 résidus d'acides aminés, et si Z₁ ou Z₂ = 0 résidu d'acide aminé, alors Z₁ = H et/ou Z₂ = COOH,
pour la fabrication d'un médicament destiné au traitement d'infections par le VIH-1 et le VIH-2.

3. Polynucléotides codant pour le peptide ou ses fragments selon la revendication 1.

4. Polynucléotides selon la revendication 3, **caractérisés en ce que** les polynucléotides sont constitués d'ADN, d'ARN, d'ADN génomique ou de PNA.

5. Vecteur contenant les polynucléotides selon la revendication 3.

6. Cellule hôte génétiquement modifiée contenant le vecteur selon la revendication 5.

7. Peptides selon la revendication 1 en combinaison avec une protéine adaptatrice qui assure l'absorption dans des cellules infectables par un virus.

8. Formulation galénique composée des peptides selon la revendication 1 ou 7 et d'un véhicule compatible.

9. Procédé de fabrication du peptide selon la revendication 1 par extraction d'hémofiltrat au moyen d'une extraction par échange de cations suivie de l'élution des substances adsorbées, puis à nouveau d'une chromatographie par échange de cations de l'extrait contenant les peptides ainsi que d'une chromatographie en phase inverse à plusieurs étapes.

10. Procédé de fabrication du peptide selon la revendication 1 par synthèse en phase solide du type synthèse de Merrifield ainsi que par synthèse en phase liquide selon les techniques connues de l'homme du métier avec des acides aminés protégés, et sa purification.

11. Procédé de fabrication du peptide selon la revendication 1. selon des procédés connus de l'homme du métier d'expression hétérologue au moyen de vecteurs biotechnologiques courants.

12. Agent diagnostique contenant les polynucléotides selon la revendication 3 ou 4.

13. Médicament contenant les peptides selon la revendication 1 ou les polynucléotides selon la revendication 3 ou 4 en tant que principe actif de formes galéniques destinées à une administration par voie orale, intraveineuse, intramusculaire, intracutanée, sous-cutanée, intrathécale et sous forme d'aérosol pour une administration par voie transpulmonaire.

14. Utilisation des polynucléotides selon la revendication 3 ou 4 pour la fabrication d'un médicament destiné au traitement d'infections par le VIH-1 ou le VIH-2.


### SEQUENZPROTOKOLL

<110> Forssmann, Wolf-Georg
   Kirchhoff, Frank
<120> Humanes zirkulierendes Virus inhibirendes Peptid (VIRIP) und seine Verwendung
<130> VIRIP
<140>
   <141>
<160> 1
<170> PatentIn Ver. 2.1
<210> 1
   <211> 20
   <212> PRT
   <213> Homo sapiens
<400> 1
   Leu Glu Ala Ile Pro Met Ser Ile Pro Pro Glu Val Lys Phe Asn Lys
     1               5                  10                  15
   Pro Phe Val Phe
                20